(19) Europäisches Patentamt
European Patent Office
Office européen des brevets

(11) Veröffentlichungsnummer: **0 602 561 A1**

# (12) EUROPÄISCHE PATENTANMELDUNG

(21) Anmeldenummer: **93119945.9**

(51) Int. Cl.$^5$: **C07C 259/06**, A01N 37/30

(22) Anmeldetag: **10.12.93**

(30) Priorität: **17.12.92 DE 4242751**

(43) Veröffentlichungstag der Anmeldung: **22.06.94 Patentblatt 94/25**

(84) Benannte Vertragsstaaten: **AT BE CH DE DK ES FR GB GR IE IT LI NL PT SE**

(71) Anmelder: **BASF Aktiengesellschaft**
**Carl-Bosch-Strasse 38**
**D-67063 Ludwigshafen(DE)**

(72) Erfinder: **Rentzea, Costin, Dr.**
**Richard-Kuhn-Strasse 1-3**
**D-69123 Heidelberg(DE)**
Erfinder: **Harreus, Albrecht Dr.**
**Teichgasse 13**
**D-67063 Ludwigshafen(DE)**
Erfinder: **Ammermann, Eberhard Dr.**
**Von-Gagern-Strasse 2**
**D-64646 Heppenheim(DE)**
Erfinder: **Lorenz, Gisela Dr.**
**Erlenweg 13**
**D-67434 Neustadt(DE)**

## (54) Oxalylhydrazid-hydroxamsäurederivate, Verfahren zu ihrer Herstellung und ihre Verwendung als Fungizide.

(57) Oxalylhydrazid-hydroxamsäure-Derivate der allgemeinen Formel I

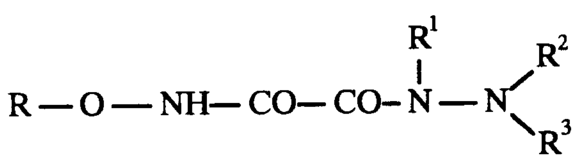

I

in der die Sustituenten folgende Bedeutung haben:

R, $R^1$, $R^2$ und $R^3$ unabhängig voneinander

Wasserstoff, gegebenenfalls substituiertes Alkyl, Alkenyl oder Alkinyl oder
gegebenenfalls substituiertes Cycloalkyl oder Cycloalkylmethyl,
gegebenenfalls substituiertes Cycloalkenyl oder Cycloalkenylmethyl,
gegebenenfalls substituiertes Phenyl, Phenylalkyl, Phenylalkenyl, Heteroaryl- oder Heteroarylalkylreste, wobei $R^2$ und $R^3$ auch zusammen mit dem Stickstoffatom, dessen Substituenten sie sind, einen 5- oder 6-gliedrigen Ring bilden können, oder
$R^2$ zusammen mit $R^3$ für die Gruppierung

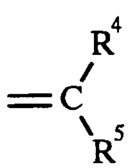

stehen, wobei $R^4$ und $R^5$ für einen der Reste R, $R^1$, $R^2$ oder $R^3$ stehen oder $R^4$ zusammen mit $R^5$ und dem C-Atom, dessen Substituenten sie sind, für monocyclisches oder polycyclisches Cycloalkyl steht, oder

EP 0 602 561 A1

$R^4$ zusammen mit $R^5$ und dem C-Atom, dessen Substituenten sie sind, einen 5- oder 6-gliedrigen, gesättigten Heterocyclus mit O, S oder -N($R^6$)- als Heterogruppierung bilden und Fungizide, die diese Wirkstoffe enthalten.

Die vorliegende Erfindung betrifft neue Oxalylhydrazid-hydroxamsäure-Derivate der allgemeinen Formel I

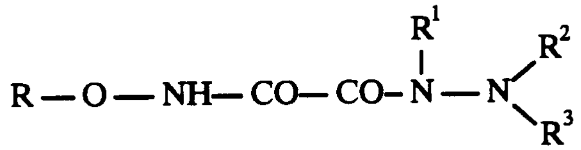

I

in der die Sustituenten folgende Bedeutung haben:
R, $R^1$, $R^2$ und $R^3$ unabhängig voneinander
Wasserstoff, $C_1$-$C_{20}$-Alkyl, $C_3$-$C_{18}$-Alkenyl oder $C_3$-$C_8$-Alkinyl, wobei diese Reste ein bis fünf Halogenatome oder ein bis drei $C_1$-$C_4$-Alkoxygruppen, gegenenfalls zusammen mit den Halogenatomen, tragen können;
monocyclisches oder polycyclisches $C_3$-$C_{10}$-Cycloalkyl oder $C_3$-$C_{10}$-cycloalkylmethyl, wobei diese Ringe ein bis drei $C_1$-$C_4$-Alkylgruppen oder einen Cyclohexylring tragen können;
monocyclisches oder polycyclisches $C_5$-$C_{10}$-Cycloalkenyl oder $C_5$-$C_{10}$-Cycloalkenylmethyl, wobei diese Ringe ein bis drei $C_1$-$C_4$-Alkylgruppen oder einen Cyclohexylring tragen können;
Phenyl, Phenyl-$C_1$-$C_4$-alkyl, Phenyl-$C_2$-$C_6$-alkenyl, einkernige Heteroaryl- oder Heteroaryl-$C_1$-$C_4$-alkylreste, wobei die (hetero)-aromatischen Reste ein bis fünf Halogenatome oder ein bis drei der folgenden Gruppen, gegebenenfalls zusammen mit den Halogenatomen tragen können: Hydroxy, Nitro, $C_1$-$C_4$-Alkyl, $C_1$-$C_4$-Halogenalkyl, $C_1$-$C_4$-Alkoxy, $C_1$-$C_4$-Halogenalkoxy, COOR, $CONH_2$ oder Amine, wobei $R^2$ und $R^3$ auch zusammen mit dem Stickstoffatom, dessen Substituenten sie sind, einen 5- oder 6-gliedrigen Ring bilden können, der noch durch Sauerstoff unterbrochen sein kann, oder
$R^2$ zusammen mit $R^3$ für die Gruppierung

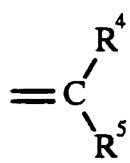

stehen, wobei $R^4$ und $R^5$ für einen der Reste R, $R^1$, $R^2$ oder $R^3$ stehen oder $R^4$ zusammen mit $R^5$ und dem C-Atom, dessen Substituenten sie sind, für monocyclisches oder polycyclisches $C_3$-$C_{10}$-Cycloalkyl steht, wobei diese Ringe ein bis drei $C_1$-$C_4$-Alkylgruppen oder einen Phenylring tragen können oder
$R^4$ zusammen mit $R^5$ und dem C-Atom, dessen Substituenten sie sind, einen 5- oder 6-gliedrigen, gesättigten Heterocyclus mit O, S oder -N($R^6$)- als Heterogruppierung bilden, wobei $R^6$ für eine $C_1$-$C_6$-Alkylgruppe steht, oder mit zwei nicht benachbarten gleichen oder verschiedenen Heteroatomen, die Sauerstoff, Schwefel oder die Gruppierung -N($R^6$) sein können, als Heterogruppierung bilden,
sowie Mittel, welche diese Oxalylhydrazid-hydroxamsäure-Derivate als Wirkstoffe enthalten, Verfahren zu ihrer Herstellung und Verfahren zur Bekämpfung von Pilzbefall auf Nutzpflanzen mit diesen Verbindungen.

Der Erfindung lag die Aufgabe zugrunde, neue Verbindungen bereitzustellen, die eine hohe fungizide Wirksamkeit aufweisen und eine gute Pflanzenverträglichkeit besitzen.

Demgemäß wurden die eingangs definierten Oxalylhydrazid-hydroxamsäure-Derivatel sowie Verfahren zu ihrer Herstellung gefunden.

Erfindungsgemäß werden Mittel, die diese Oxalylderivate als Wirkstoff enthalten und ein Verfahren zur Bekämpfung von phytopathogen Pilzen in Nutzpflanzenkulturen mit diesen Verbindungen bereitgestellt.

Im einzelnen haben die Reste $R^1$ bis $R^5$ in Formel I die folgende Bedeutung:
R, $R^1$, $R^2$ und $R^3$ sind gleich oder verschieden und können breit variiert werden.

Beispielsweise stehen sie für Wasserstoff, geradkettiges oder verzweigtes $C_1$- bis $C_{20}$-Alkyl, insbesondere $C_1$- bis $C_{16}$-Alkyl wie Methyl, Ethyl, n-Propyl, iso-Propyl, n-Butyl, iso-Butyl, sek.-Butyl, Pentyl, Hexyl, Heptyl, Octyl, Nonyl, Decyl, Undecyl, Tridecyl, Tetradecyl, Pentadecyl, Hexadecyl, Heptadecyl, Octadecyl, Nonadecyl; für geradkettiges oder verzweigtes $C_1$-$C_{20}$-Halogenalkyl, insbesondere $C_1$-$C_{12}$-Halogenalkyl mit 1 bis 5, beispielsweise 1 bis 3, vorzugsweise 1 bis 2 Halogenatomen wie Jod, Brom, insbesondere Fluor oder Chlor wie Chlorbutyl, z. B. 1-Chlorbutyl, 2-Chlorbutyl, 3-Chlorbutyl, 4-Chlorbutyl, 2,3-Dichlorbutyl, Chlorhexyl, z. B. 3-Chlorhexyl, 5-Chlorhexyl, Fluorpentyl, z. B. 3-Fluorpentyl, Fluorhexyl, Fluorheptyl, Fluordecyl, Fluordodecyl; für $C_3$-$C_{18}$-Alkenyl, insbesondere $C_3$-$C_{16}$-Alkenyl mit 1 bis 3 Doppelbindungen im

Alkenylrest wie Allyl, Methallyl, Pentenyl, Hexenyl, Heptenyl, Octenyl, 2-Chlorallyl, 2-Bromallyl, 3-Chlorallyl, 3-Bromallyl, 2,3,3-Trichlorallyl, 3-Chlor-2-butenyl, Decenyl, Dodecenyl, Geranyl, Hexadecenyl, Octadecenyl; für $C_3$- oder $C_4$-Alkinyl wie Propargyl oder 2-Butinyl; für ggf. überbrücktes, d. h. 1 oder 2 Methylen- oder Ethylenbrücken tragendes $C_3$-$C_{10}$-Cycloalkyl oder $C_3$-$C_{10}$-Cycloalkylmethyl, insbesondere $C_3$-$C_8$-Cycloalkyl bzw. $C_3$-$C_8$-Cycloalkylmethyl, wobei die genannten Cycloalkyl- bzw. Cycloalkyl-alkyl-reste $C_1$-$C_5$-alkylsubstituiert oder cyclohexylsubstituiert sein können. Beispielsweise seien die folgenden Reste aufgeführt: Cycloalkylreste wie Cyclopropyl, Cyclopentyl, Methylcyclopentyl, Cyclohexyl, Ethylcyclohexyl, Propyl- und Isopropylcyclohexyl, Butyl-, Isobutyl-, sek.-Butyl- und tert.-Butylcyclohexyl, tert.-Amylcyclohexyl, Cyclohexylcyclohexyl, Cycloheptyl, Methylcycloheptyl, Propylcycloheptyl, Cyclooctyl, Cyclododecyl, Decalyl (Decahydronaphthyl); Cycloalkylmethylreste, wie Cyclopropylmethyl, Cyclopentylmethyl, Cyclohexylmethyl, Cyclododecylmethyl; überbrückte Cycloalkyl- bzw. Cycloalkylmethylreste wie Norbornyl, 1,5-Dimethylbicyclo-[2.3.1]octan-8-yl, Tricyclodecanyl, Norbornylmethyl, Adamantyl sowie mono- und bicyclische Terpenreste wie o-Menthyl, m-Menthyl, p-Menthyl, Bornyl, Isobornyl, Pinanyl, Camphenyl und Homocamphenyl.

Weiterhin stehen die Reste R, $R^1$, $R^2$ und $R^3$ für Phenyl, Phenyl-$C_1$-$C_4$-alkyl, Phenyl-$C_2$-$C_6$-alkenyl, für einkernige Heteroaryl- oder Heteroaryl-$C_1$-$C_4$-alkylreste, die mit Halogen, Hydroxy, Nitro, $C_1$-$C_4$-Alkyl-, $C_1$-$C_4$-Halogenalkyl-, $C_1$-$C_4$-Alkoxy-, $C_1$-$C_4$-Halogenalkoxy-, COOR-, $CONH_2$- oder Aminogrupppen substituiert sein können, insbesondere Phenyl, Phenylmethyl, Phenylethyl, Phenylpropyl, 2-Fluorphenyl, 3-Fluorphenyl, 4-Fluorphenyl, 2-Chlorphenyl, 3-Chlorphenyl, 4-Chlorphenyl, 4-Bromphenyl, 2-, 3-Dichlorphenyl, 2,4-Dichlorphenyl, 3,4-Dichlorphenyl, 2-Hydroxyphenyl, 3-Hydroxyphenyl, 4-Hydroxyphenyl, 2-Hydroxy-3-chlorphenyl, 2,6-Dichlorphenyl, 2-Hydroxy-5-chlorphenyl, 2-Hydroxy-2,5-dichlorphenyl, 2-Hydroxy-3-bromphenyl, 2-Hydroxy-2,5-dibromphenyl, 4-Hydroxy-3,5-dichlorphenyl, 4-Hydroxy-3,5-dibromphenyl, 2-Hydroxy-3-methoxyphenyl, 2-Hydroxy-4-methoxyphenyl, 2-Hydroxy-5-methoxyphenyl, 3-Methoxy-4-hydroxyphenyl, 4-Hydroxy-3-methoxyphenyl, 2-Methylphenyl, 3-Methylphenyl, 4-Methylphenyl, 2,4-Dimethylphenyl, 2,5-Dimethylphenyl, 2,6-Dimethylphenyl, 4-iso-Propylphenyl, 4-tert.-Butylphenyl, 4-sek.-Butylphenyl, 2-Trifluormethylphenyl, 3-Trifluormethylphenyl, 4-Trifluormethylphenyl, 4-Nitrophenyl, 2-Hydroxy-3-nitrophenyl, 2-Hydroxy-5-nitrophenyl, 2-Methoxyphenyl, 3-Methoxyphenyl, 4-Methoxyphenyl, 4-Ethoxyphenyl, 4-Butoxyphenyl, 2,3-Dimethoxyphenyl, 2,4-Dimethoxyphenyl, 2,5-Dimethoxyphenyl, 3,4-Dimethoxyphenyl, 3,5-Dimethoxyphenyl, 2,3,4-Trimethoxyphenyl, 3,4,5-Trimethoxyphenyl, 2-Fluorbenzyl, 3-Fluorbenzyl, 4-Fluorbenzyl, 2-Chlorbenzyl, 3-Chlorbenzyl, 4-Chlorbenzyl, 4-Brombenzyl, 2,4-Dichlorbenzyl, 2,6-Dichlorbenzyl, 3,4-Dichlorbenzyl, 4-Nitrobenzyl, 2-Methylbenzyl, 3-Methylbenzyl, 4-Methylbenzyl, 2,4-Dimethylbenzyl, 3,4-Dimethylbenzyl, 2,5-Dimethylbenzyl, 4-iso-Propylbenzyl, 4-sek.-Butylbenzyl, 4-tert.-Butylbenzyl, 4-Methoxybenzyl, 4-Carboxyethylbenzyl, 2-Trifluormethylbenzyl, 3-Trifluormethylbenzyl, 4-Trifluormethylbenzyl, 2-Thienyl, 3-Thienyl, 2-Furanyl, 3-Furanyl, 3-Isoxazolyl, 4-Isoxazolyl, 5-Isoxazolyl, 2-Thiazolyl, 2-Thienylmethyl, 5-Chlor-2-thienyl, 5-Chor-2-thienylmethyl, 5-Brom-2-thienyl, 5-Chlor-3-thienyl, 5-Brom-3-thienyl, 5-Methyl-2-thienyl, 5-Methyl-2-thienylmethyl, 2-Furanylmethyl, 5-Methyl-2-furanyl, 5-Methyl-2-furanylmethyl, 5-Nitro-2-furanyl, 5-Nitro-2-thienyl, 3-Isoxazolylmethyl, 4-Isoxazolylmethyl, 5-Isoxazolylmethyl, 2-Pyridinyl, 3-Pyridinyl, 4-Pyridinyl, 2-Pyridinylmethyl, 3-Pyridinylmethyl, 4-Pyridinylmethyl.

Weiterhin stehen $R^4$ zusammen mit $R^5$ und dem C-Atom, dessen Substituenten sie sind, auch für N-Methyl-piperidinylen-, für N-Methylpyrrolidinylen-, für Tetrahydropyranyliden-, für Tetrahydrofuranyliden-, für Hexahydrooxazinyliden-, für Hexahydrothiazinyliden- oder für Dithianylidenreste.

Die neuen Verbindungen lassen sich herstellen, indem man

a) Hydrazine der Formel II

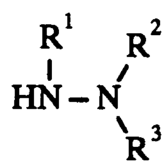

(II)

mit einem Oxalyl-hydroxamester der Formel III

R-O-NH-CO-$COOR^6$ (III)

wobei R, $R^1$, $R^2$, $R^3$ und $R^6$ die oben genannte Bedeutung haben, oder

b) ein Oxalylhydrazid-hydroxamsäureester der Formel IV

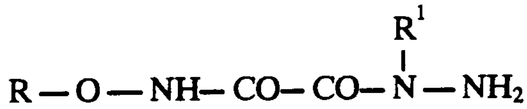 (IV)

mit einem Aldehyd oder Keton der Formel V

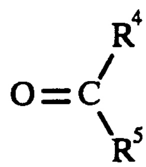 (V)

wobei R, $R^1$, $R^4$ und $R^5$ die oben angegebenen Bedeutungen haben, gegebenenfalls in Gegenwart eines Lösungs- oder Verdünnungsmittels oder einer anorganischen oder organischen Base oder einer anorganischen oder organischen Säure oder eines Reaktionsbeschleunigers oder deren Mischungen umsetzt.

Als Lösungsmittel für die Verfahrensvariante a) eignen sich beispielsweise Halogenkohlenwasserstoffe, insbesondere Chlorkohlenwasserstoffe, z. B. 1,1,2,2-Tetrachlorethylen, oder 1,1,2,2-Tetrachlorethan, Dichlorpropan, Methylenchlorid, Dichlorbutan, 1,1,1- oder 1,1,2-Trichlorethan, Trichlorethylen, o-, m-, p-Difluorbenzol, 1,2-Dichlorethan, 1,1-Dichlorethan, 1,2-cis-Dichlorethylen, Chlorbenzol, Fluorbenzol, Brombenzol, Jodbenzol, o-, m-, p-Dichlorbenzol, o-, p-, m-Dibrombenzol, o-, m-, p-Chlortoluol; Ether, z. B. Ethylpropylether, Methyl-tert.-butylether, n-Butylethylether, Di-n-Butylether, Di-isobutylether, Diisoamylether, Diisopropylether, Anisol, Phenethol, Cyclohexylmethylether, Diethylether, Ethylenglykoldimethylether, Tetrahydrofuran, Dioxan, Thioanisol; Alkohole wie Methanol, Ethanol, Propanol, iso-Propanol, n-Butanol, iso-Butanol, tert.-Butanol, Ethylenglykol und Propandiole; Nitrile wie Acetonitril, Butyronitril, Isobutyronitril, Benzonitril, m-Chlorbenzonitril; aliphatische oder cycloaliphatische Kohlenwasserstoffe, z. B. Heptan, Pinan, Nonan, o-, m-, p-Cymol, Benzinfraktionen innerhalb eines Siedepunktintervalles von 70 bis 190 °C, Cyclohexan, Methylcyclohexan, Dekalin, Petrolether, Hexan, Ligroin, 2,2,4-Trimethylpentan, 2,2,3-Trimethylpentan, 2,3,3-Trimethylpentan, Octan; Ester z. B. Ethylacetat, Acetessigester, Isobutylacetat; Amide, z. B. Formamid, Methylformamid, Dimethylformamid, gegebenenfalls auch Wasser und entsprechende Gemische in Betracht. Als Lösungsmittel können auch die Verbindungen der Formel I im Überschuß verwendet werden. Zweckmäßig verwendet man das Lösungsmittel in einer Menge von 100 bis 2000Gew.-%, vorzugsweise von 200 bis 700Gew.-%, bezogen auf Ausgangsstoff III.

Als Basen kommen beispielsweise Kaliumhydroxid, Natriumhydroxid, Kaliumcarbonat, Natriumcarbonat, Lithiumhydroxid, Lithiumcarbonat, Natriumbicarbonat, Kaliumbicarbonat, Calciumhydroxid, Calciumoxid, Bariumoxid, Magnesiumhydroxid, Magnesiumoxid, Bariumhydroxid, Cycliumcarbonat, Magnesiumcarbonat, Magnesiumbicarbonat, Magnesiumacetat, Zinkhydroxid, Zinkoxid, Zinkcarbonat, Zinkbicarbonat, Zinkacetat, Natriumformiat, Natriumacetat, Trimethylamin, Triethylamin, Tripropylamin, Triisopropylamin, Tributylamin, Triisobutylamin, Tri-sec.-butylamin, Tri-tert.-butylamin, Tribenzylamin, Tricyclohexylamin, Triamylamin, Trihexylamin, N,N-Dimethylanilin, N,N-Diethylanilin, N,N-Dipropylanilin, N,N-Dimethyltoluidin, N,N-Diethyltoluidin, N,N-Dipropyltoluidin, N,N-Dimethyl-4-aminopyridin, N,N-Diethyl-4-aminopyridin, N,N-Dipropyl-4-aminopyridin, N,N-Dipropyl-4-aminopyridin, N-Methylpyrrolidin, N-Ethylpyrrolidin, N-Methylpiperidin, N-Ethylpiperidin, N-Methyl-pyrrolidin, N-Ethyl-pyrrolidin, N-Methylimidazol, N-Ethylimidazol, N-Methylmorpholin, N-Ethylmorpholin, N-Methylhexamethylenimin, N-Ethylhexamethylenimin, Pyridin, Chinolin, alpha-Picolin, beta-Picolin, Isochinolin, Pyrimidin, Acridin, N,N,N',N'-Tetramethylethylendiamin, N,N,N',-N'-Tetraethylethylendiamin, Chinoxalin, N-Propyldiisopropylamin, N,N-Dimethylcyclohexylamin, 2,6-Lutidin, 2,4-Lutidin, Triethylendiamin in Betracht.

Zweckmäßig verwendet man die Base in stöchiometrischen Mengen, im Überschuß oder Unterschuß von jeweils bis zu 20 Mol.-%, bezogen auf den Ausgangsstoff III.

Außerdem kann es von Nutzen sein, die Umsetzung in Gegenwart eines Katalysators durchzuführen.

Als Reaktionsbeschleuniger kommen vorzugsweise 4-Dimethylaminopyridin und 4-Pyrrolidinopyridin in Betracht (J. Cossy et al., Synthesis, 753f (1989)).

Ausgangsstoffe der Formel III sind aus DE 4 022 265 bekannt.

Für die Verfahrensvariante b) erfolgt die Umsetzung im allgemeinen bei Temperaturen von -20 bis 100 °C, vorzugsweise von 15 bis 80 °C.

Als Lösungsmittel eignen sich beispielsweise die vorstehend bei Verfahren a) genannten. Insbesondere kommen die folgenden in Betracht: Essigsäure, Essigsäureethylester, Methylenchlorid, Toluol, Chlorbenzol, Tetrahydrofuran, Dioxan, Methanol, Ethanol, n-Propanol, iso-Propanol, n-Butanol, iso-Butanol und tert.-Butanol oder deren Gemische.

Als Basen kommen in diesem Verfahren neben den vorstehend genannten Kaliumacetat und Natriumacetat in Betracht.

Im einzelnen seien außer den bei den Herstellungsbeispielen genannten Verbindungen die folgenden Oxalylhydrazidhydroxamsäure-Derivate der allgemeinen Formel I genannt:

Tabelle 1

| R | $R^1$ | $R^2$ | $R^3$ |
|---|---|---|---|
| H | H | H | H |
| $CH_3$ | H | H | H |
| $CH_3$ | $CH_3$ | H | H |
| $CH_3$ | $CH_3$ | $CH_3$ | H |
| $CH_3$ | H | $CH_3$ | $CH_3$ |
| $CH_3$ | $C_2H_5$ | H | H |
| $CH_3$ | n-$C_3H_7$ | H | H |
| $CH_3$ | iso-$C_3H_7$ | H | H |
| $CH_3$ | H | -C($CH_3$) | H |
| $CH_3$ | n-$C_4H_9$ | H | H |
| $CH_3$ | iso-$C_4H_9$ | H | H |
| $CH_3$ | n-$C_5H_{11}$ | H | H |
| $CH_3$ | iso-$C_5H_{11}$ | H | H |
| $CH_3$ | n-$C_6H_{13}$ | H | H |
| $CH_3$ | n-$C_8H_{17}$ | H | H |
| $CH_3$ | n-$C_{10}H_{21}$ | H | H |
| $CH_3$ | Cyclopropyl | H | H |
| $CH_3$ | Cyclopentyl | H | H |
| $CH_3$ | Cyclohexyl | H | H |
| $CH_3$ | 4-Methylcyclohexyl | H | H |

Tabelle 1 (Fortsetzung)

| R | $R^1$ | $R^2$ | $R^3$ |
|---|---|---|---|
| $CH_3$ | Cyclohexyl-methyl | H | H |
| $CH_3$ | Cycloheptyl | H | H |
| $CH_3$ | $-CH_2CH_2-Cl$ | H | H |
| $CH_3$ | $-CH_2CH_2CH_2Cl$ | H | H |
| $CH_3$ | $-(CH_2)_4Cl$ | H | H |
| $CH_3$ | $-(CH_2)_5Cl$ | H | H |
| $CH_3$ | $-CH_2-CF_3$ | H | H |
| $CH_3$ | $-(CH_2)_5Br$ | H | H |
| $CH_3$ | $-CH_2-CH_2OCH_3$ | H | H |
| $CH_3$ | $-(CH_2)_3OCH_3$ | H | H |
| $CH_3$ | $-(CH_2)_2OC_2H_5$ | H | H |
| $CH_3$ | $-(CH_2)_3OC_2H_5$ | H | H |
| $CH_3$ | $-(CH_2)_4OCH_3$ | H | H |
| $CH_3$ | $-CH_2CH=CH_2$ | H | H |
| $CH_3$ | $-CH_2-CH=CH-CH_3$ | H | H |
| $CH_3$ | $-CH_2-CH=CH(CH_3)_2$ | H | H |
| $CH_3$ | $-CH_2CH=CH-C_2H_5$ | H | H |
| $CH_3$ | $-CH_2CH=CH-C_3H_7$ | H | H |
| $CH_3$ | $-CH_2C(Cl)=CH_2$ | H | H |
| $CH_3$ | $-CH_2C(Br)=CH_2$ | H | H |
| $CH_3$ | $-CH_2CH=CHCl$ | H | H |

Tabelle 1 (Fortsetzung)

| R | $R^1$ | $R^2$ | $R^3$ |
|---|---|---|---|
| $CH_3$ | $-CH_2CH{=}CHBr$ | H | H |
| $CH_3$ | H | $-C_6H_5$ | H |
| $CH_3$ | H | $-C_6H_5$ | $CH_3$ |
| $CH_3$ | H | $-C_6H_4-4F$ | H |
| $CH_3$ | H | $-C_6H_4-4Cl$ | H |
| $CH_3$ | H | $-C_6H_4-2Cl$ | H |
| $CH_3$ | H | $-C_6H_4-4Br$ | H |
| $CH_3$ | H | $-C_6H_4-2CH_3$ | H |
| $CH_3$ | H | $-C_6H_4-3CH_3$ | H |
| $CH_3$ | H | $-C_6H_4-4CH_3$ | H |
| $CH_3$ | H | $-C_6H_4-4CF_3$ | H |
| $CH_3$ | H | $-C_6H_4-4NO_2$ | H |
| $CH_3$ | H | $-C_6H_4-4OCH_3$ | H |
| $CH_3$ | H | $-C_6H_4-4C(CH_3)_3$ | H |
| $CH_3$ | H | $-C_6H_4-4(OC_2H_5)$ | H |
| $CH_3$ | H | $-C_6H_4-4-N(CH_3)_2$ | H |
| $CH_3$ | H | $-C_6H_4-4-CONH_2$ | H |
| $CH_3$ | H | $-C_6H_3-2,4Cl_2$ | H |
| $CH_3$ | H | $-C_6H_3-3,4Cl_2$ | H |
| | | $R^2 + R^3$ | |
| $CH_3$ | H | $=CH-CH_3$ | |
| $CH_3$ | H | $=CH-C_2H_5$ | |

Tabelle 1 (Fortsetzung)

| R | $R^1$ | $R^2 + R^3$ |
|---|---|---|
| $CH_3$ | H | $=CH-C_3H_7-n$ |
| $CH_3$ | H | $=CH-C_3H_7-iso$ |
| $CH_3$ | H | $=CH-C_4H_9-n$ |
| $CH_3$ | H | $=CH-C_4H_9-iso$ |
| $CH_3$ | H | $=CH-C_4H_9-tert$ |
| $CH_3$ | H | $=CH-C_5H_{11}-n$ |
| $CH_3$ | H | $=CH-C_5H_{11}-iso$ |
| $CH_3$ | H | $=CH-C_6H_{13}-n$ |
| $CH_3$ | H | $=CH-C_7H_{15}-n$ |
| $CH_3$ | H | $=CH-C_8H_{17}-n$ |
| $CH_3$ | H | $=CH-C_9H_{19}-n$ |
| $CH_3$ | H | $=C(CH_3)_2$ |
| $CH_3$ | H | $=C(CH_3)C_2H_5$ |
| $CH_3$ | H | $=C(CH_3)C_3H_7-n$ |
| $CH_3$ | H | $=C(C_2H_5)_2$ |
| $CH_3$ | H | $=C(C_3H_7)_2$ |
| $CH_3$ | H | $=C(CH_3)C_4H_9-n$ |
| $CH_3$ | H | $=C(CH_3)C_4H_9-iso$ |
| $CH_3$ | H | $=C(CH_3)C_4H_9-tert$ |
| $CH_3$ | H | $=C(CH_3)C_5H_{11}-n$ |
| $CH_3$ | H | $=C(CH_3)C_6H_{13}-n$ |
| $CH_3$ | H | $=C(CH_3)C_7H_{15}-n$ |

Tabelle 1 (Fortsetzung)

| R | $R^1$ | $R^2$ + $R^3$ |
|---|---|---|
| $CH_3$ | H | $=C(CH_3)C_8H_{17}-n$ |
| $CH_3$ | H | $=C(C_4H_9)C_6H_{13}-n$ |
| $CH_3$ | H | 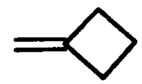 |
| $CH_3$ | H | 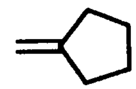 |
| $CH_3$ | H | 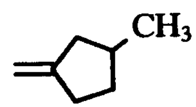 |
| $CH_3$ | H | 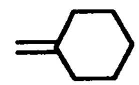 |
| $CH_3$ | H | 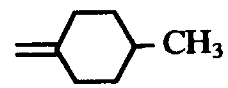 |
| $CH_3$ | H | 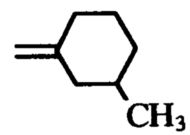 |

Tabelle 1 (Fortsetzung)

| R | $R^1$ | $R^2$ + $R^3$ |
|---|---|---|
| $CH_3$ | H | 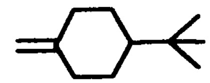 |
| $CH_3$ | H | 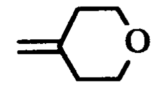 |
| $CH_3$ | H | 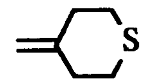 |
| $CH_3$ | H | 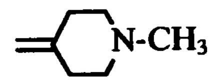 |
| $CH_3$ | H | 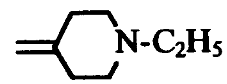 |
| $CH_3$ | H | 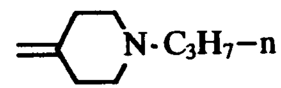 |
| $CH_3$ | H | $=CH-CH=CH_2$ |
| $CH_3$ | H | $=CH-CH=CH-CH_3$ |
| $CH_3$ | H | $=CH-CH=C(CH_3)_2$ |
| $CH_3$ | H | $=CH-C_6H_5$ |
| $CH_3$ | H | $=CH-CH_2-C_6H_5$ |

Tabelle 1 (Fortsetzung)

| R | $R^1$ | $R^2$ + $R^3$ |
|---|---|---|
| $CH_3$ | H | $=CH-CH_2CH_2-C_6H_5$ |
| $CH_3$ | H | $=CH-CH(CH_3)CH_2C_6H_5$ |
| $CH_3$ | H | $=CH-CH=CH-C_6H_5$ |
| $CH_3$ | H | $=CH-C_6H_4-2F$ |
| $CH_3$ | H | $=CH-C_6H_4-3F$ |
| $CH_3$ | H | $=CH-C_6H_4-4F$ |
| $CH_3$ | H | $=CH-C_6H_4-2Cl$ |
| $CH_3$ | H | $=CH-C_6H_4-3Cl$ |
| $CH_3$ | H | $=CH-C_6H_4-4Cl$ |
| $CH_3$ | H | $=CH-C_6H_3-2OH-5Cl$ |
| $CH_3$ | H | $=CH-C_6H_3-2OH-3Cl$ |
| $CH_3$ | H | $=CH-C_6H_2-2OH-3,5Cl_2$ |
| $CH_3$ | H | $=CH-C_6H_4-2OH$ |
| $CH_3$ | H | $=CH-C_6H_4-3OH$ |
| $CH_3$ | H | $=CH-C_6H_4-4OH$ |
| $CH_3$ | H | $=CH-C_6H_4-4Br$ |
| $CH_3$ | H | $=CH-C_6H_3-2OH-3Br$ |
| $CH_3$ | H | $=CH-C_6H_3-2OH-5Br$ |
| $CH_3$ | H | $=CH-C_6H_2-2OH-3,5Br_2$ |
| $CH_3$ | H | $=CH-C_6HH_3-3Br-4OH$ |
| $CH_3$ | H | $=CH-C_6H_3-2,4-Cl_2$ |
| $CH_3$ | H | $=CH-C_6H_3-3,4-Cl_2$ |

Tabelle 1 (Fortsetzung)

| R | $R^1$ | $R^2$ + $R^3$ |
|---|---|---|
| $CH_3$ | H | $=CH-C_6H_4-4CH_3$ |
| $CH_3$ | H | $=CH-C_6H_4-4C_3H_7$-iso |
| $CH_3$ | H | $=CH-C_6H_4-4C_4H_9$-tert |
| $CH_3$ | H | $=CH-C_6H_4-4-C_6H_{13}$-n |
| $CH_3$ | H | $=CH-C_6H_4-2OCH_3$ |
| $CH_3$ | H | $=CH-C_6H_4-3OCH_3$ |
| $CH_3$ | H | $=CH-C_6H_4-4OCH_3$ |
| $CH_3$ | H | $=CH-C_6H_3-2,4(OCH_3)_2$ |
| $CH_3$ | H | $=CH-C_6H_3-2,5(OCH_3)_2$ |
| $CH_3$ | H | $=CH-C_6H_3-3,4(OCH_3)_2$ |
| $CH_3$ | H | $=CH-C_6H_3-2,3(OCH_3)_2$ |
| $CH_3$ | H | $=CH-C_6H_3-3OH-4OCH_3$ |
| $CH_3$ | H | $=CH-C_6H_4-4CF_3$ |
| $CH_3$ | H | $=CH-C_6H_4-4NO_2$ |
| $CH_3$ | H | $=CH-C_6H_3-2OH-5NO_2$ |
| $CH_3$ | H | $=CH-C_6H_4-4-COOC_2H_5$ |
| $CH_3$ | H | $=CH-C_6H_4-4-CONH_2$ |
| $CH_3$ | H | $=CH-C_6H_4-4-N(CH_3)_2$ |
| $CH_3$ | H | =CH-(Thienyl-2) |
| $CH_3$ | H | =CH-(Thienyl-3) |
| $CH_3$ | H | =CH-(5-Chlor-thienyl-2) |
| $CH_3$ | H | =CH-(5-Brom-thienyl-2) |

Tabelle 1 (Fortsetzung)

| R | $R^1$ | $R^2$ + $R^3$ | |
|---|---|---|---|
| $CH_3$ | H | =CH-(5-Chlor-thienyl-3) | |
| $CH_3$ | H | =CH-(5-Brom-thienyl-3) | |
| $CH_3$ | H | =CH-(5-Methyl-thienyl-2) | |
| $CH_3$ | H | =CH-(5-Mehtyl-thienyl-3) | |
| $CH_3$ | H | =CH-(Furanyl-2) | |
| $CH_3$ | H | =CH-(5-Methyl-furanyl-2) | |
| $CH_3$ | H | =CH-(Pyridinyl-2) | |
| $CH_3$ | H | =CH-(Pyridinyl-3) | |
| $CH_3$ | H | =CH-(Pyridinyl-4) | |
| $CH_3$ | H | =CH-(Isoxazolyl-3) | |
| $CH_3$ | H | =CH-(Isoxazolyl-4) | |
| $CH_3$ | H | =CH-(Isoxazolyl-5) | |
| $CH_3$ | H | =CH-(5-Methyl-isoxazolyl-3) | |
| | | $R^2$ | $R^3$ |
| $CH_3$ | $-CH_2-C_6H_5$ | H | H |
| $CH_3$ | $-CH_2CH_2-C_6H_5$ | H | H |
| $CH_3$ | $-(CH_3)_3-C_6H_5$ | H | H |
| $CH_3$ | $-CH_2-C_6H_5-4F$ | H | H |
| $CH_3$ | $-CH_2-C_6H_4-2F$ | H | H |
| $CH_3$ | $-CH_2-C_6H_4-2Cl$ | H | H |
| $CH_3$ | $-CH_2-C_6H_4-4Cl$ | H | H |
| $CH_3$ | $-CH_2-C_6H_4-4CH_3$ | H | H |

Tabelle 1 (Fortsetzung)

| R | $R^1$ | $R^2$ | $R^3$ |
|---|---|---|---|
| $CH_3$ | $-CH_2-C_6H_4-4OCH_3$ | H | H |
| $CH_3$ | $-CH_2C_6H_4-4C(CH_3)_3$ | H | H |
| $CH_3$ | $-CH_2C_6H_4-4OC_2H_5$ | H | H |
| $CH_3$ | $-CH_2C_6H_4-4OC_3H_7$ | H | H |
| $CH_3$ | $-CH_2C_6H_4-4CF_3$ | H | H |
| $CH_3$ | $-CH_2-C_6H_3-2,4-Cl_2$ | H | H |
| $C_2H_5$ | H | H | H |
| $C_2H_5$ | $CH_3$ | H | H |
| $C_2H_5$ | $CH_3$ | $CH_3$ | H |
| $C_2H_5$ | H | $CH_3$ | $CH_3$ |
| $C_2H_5$ | $C_2H_5$ | H | H |
| $C_2H_5$ | iso-$C_3H_7$ | H | H |
| $C_2H_5$ | n-$C_3H_7$ | H | H |
| $C_2H_5$ | H | $-C(CH_3)_3$ | H |
| | | $R^2 + R^3$ | |
| $C_2H_5$ | H | $=CH-C_4H_9$-n | |
| $C_2H_5$ | H | $=CH-C_5H_{11}$-n | |
| $C_2H_5$ | H | $=CH-C_9H_{19}$-n | |
| $C_2H_5$ | H | $=CH-C_6H_5$ | |
| $C_2H_5$ | H | $=CH-C_6H_4-2OH$ | |
| $C_2H_5$ | H | $=CH-C_6H_3-2OH-3Cl$ | |
| $C_2H_5$ | H | $=CH-C_6H_3-2OH-5Cl$ | |

Tabelle 1 (Fortsetzung)

| R | $R^1$ | $R^2$ | $R^3$ | |
|---|---|---|---|---|
| $C_2H_5$ | H | $=CH-C_6H_2-2OH-3,5Cl_2$ | | Fp 330°C |
| $C_2H_5$ | H | $=CH-C_6H_4-4Cl$ | | |
| $C_2H_5$ | H | $=CH-CH=CH-C_6H_5$ | | |
| $C_2H_5$ | H | $=CH-CH=CH-C_6H_4-4OCH_3$ | | |
| $C_2H_5$ | H | 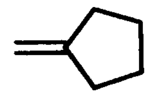 | | |
| $C_2H_5$ | H | 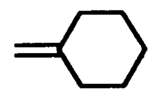 | | |
| $C_2H_5$ | H | 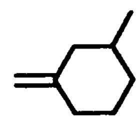 | | |
| $C_2H_5$ | H | 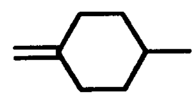 | | |
| $C_2H_5$ | H | $C_6H_5$ | H | |
| $C_2H_5$ | H | $C_6H_5$ | $CH_3$ | |
| $C_3H_7-n$ | H | H | H | |
| $C_3H_7-n$ | $CH_3$ | H | H | |

Tabelle 1 (Fortsetzung)

| R | $R^1$ | $R^2$ | $R^3$ |
|---|---|---|---|
| $C_3H_7$-n | $CH_3$ | $CH_3$ | H |
| $C_3H_7$-n | H | $CH_3$ | $CH_3$ |
| $C_3H_7$-n | iso-$C_3H_7$ | H | H |
| $C_3H_7$-n | H | $C_6H_5$ | H |
| $C_3H_7$-n | H | $C_6H_5$ | $CH_3$ |
| | | $R^2$ + $R^3$ | |
| $C_3H_7$-n | H | 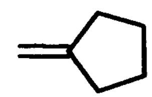 | |
| $C_3H_7$-n | H | 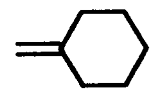 | |
| $C_3H_7$-n | H | 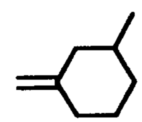 | |
| $C_3H_7$-n | H | 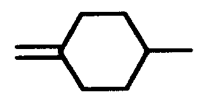 | |
| $C_3H_7$-n | H | 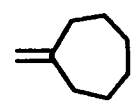 | |
| $C_3H_7$-iso | H | H | H |

Tabelle 1 (Fortsetzung)

| R | $R^1$ | $R^2$ + | $R^3$ |
|---|---|---|---|
| $C_3H_7$-n | H | $=CH-C_9H_{19}$-n | |
| $C_3H_7$-n | H | $=CH-C_{10}H_{21}$-n | |
| $C_3H_7$-n | H | $=CH-C_{13}H_{27}$-n | |
| $C_3H_7$-n | H | $=CH-C_6H_5$ | |
| $C_3H_7$-n | H | $=CH-C_6H_4$-2OH | |
| $C_3H_7$-n | H | $=CH-C_6H_4$-3OH | |
| $C_3H_7$-n | H | $=CH-C_6H_4$-4OH | |
| $C_3H_7$-n | H | $=CH-C_6H_3$-2OH-3Cl | |
| $C_3H_7$-n | H | $=CH-C_6H_3$-2OH-5Cl | |
| $C_3H_7$-n | H | $=CH-C_6H_2$-2OH-3,5$Cl_2$ | |
| $C_3H_7$-n | H | $=CH-C\equiv CH$ | |
| $C_3H_7$-n | H | $=CH-C\equiv C-CH_3$ | |
| | | $R^2$ | $R^3$ |
| $C_4H_9$-n | H | H | H |
| $C_4H_9$-n | $CH_3$ | H | H |
| $C_4H_9$-n | $CH_3$ | $CH_3$ | H |
| $C_4H_9$-n | H | $CH_3$ | $CH_3$ |
| $C_4H_9$-n | iso-$C_3H_7$ | H | H |
| $C_4H_9$-n | iso-$C_3H_7$ | $=CH-C_6H_4$-2OH | |
| $C_4H_9$-n | iso-$C_3H_7$ | $=CH-CH=CH-C_6H_4-2OCH_3$ | |
| $C_4H_9$-n | H | $-C_6H_5$ | H |
| $C_4H_9$-n | H | $-CH_2-C_6H_5$ | H |

Tabelle 1 (Fortsetzung)

| R | $R^1$ | $R^2$ + | $R^3$ |
|---|---|---|---|
| $C_4H_9$-n | H | $=CH-C_{15}H_{31}$-n | |
| $C_4H_9$-n | H | $=CH-C_{17}H_{35}$-n | |
| $C_4H_9$-n | H | $=CH-C_6H_3$-2OH-3Cl | |
| $C_4H_9$-n | H | $=CH-C_6H_3$-2OH-5Cl | |
| $C_4H_9$-n | H | 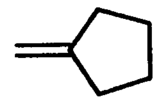 | |
| $C_4H_9$-n | H | 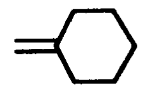 | |
| $C_4H_9$-n | H | 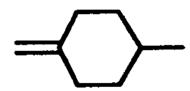 | |
| | | $R^2$ | $R^3$ |
| $C_4H_9$-iso | H | H | H |
| $C_4H_9$-iso | $CH_3$ | H | H |
| $C_4H_9$-iso | H | $CH_3$ | H |
| $C_4H_9$-iso | $CH_3$ | $CH_3$ | H |
| $C_4H_9$-iso | H | $CH_3$ | $CH_3$ |
| $C_4H_9$-iso | $C_2H_5$ | H | H |
| $C_4H_9$-iso | $C_3H_7$-iso | H | H |
| $C_4H_9$-iso | H | $-C(CH_3)_3$ | H |

Tabelle 1 (Fortsetzung)

| R | $R^1$ | $R^2$ | $R^3$ |
|---|---|---|---|
| $C_4H_9$-iso | H | $-C_6H_5$ | H |
| $C_4H_9$-iso | H | $-C_6H_5$ | $CH_3$ |
| $C_4H_9$-iso | H | $-C_6H_4$-4F | H |
| $C_4H_9$-iso | H | $-C_6H_4$-4Cl | H |
| $C_4H_9$-iso | H | $-C_6H_4$-4$CH_3$ | H |
| $C_4H_9$-iso | H | $-C_6H_4$-4$N(CH_3)_2$ | H |
| $C_4H_9$-iso | H | $=CH-C_3H_7$-n | |
| $C_4H_9$-iso | H | $=CH-C_4H_9$-iso | |
| $C_4H_9$-iso | H | $=CH-C_5H_{11}$-n | |
| $C_4H_9$-iso | H | $=CH-C_9H_{19}$-n | |
| $C_4H_9$-iso | H | $=C(CH_3)C_3H_7$ | |
| | | $R^2 + R^3$ | |
| $C_4H_9$-iso | H | 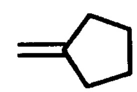 | |
| $C_4H_9$-iso | H | 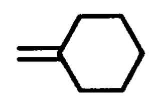 | |
| $C_4H_9$-iso | H | 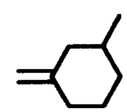 | |

Tabelle 1 (Fortsetzung)

| R | $R^1$ | $R^2$ + $R^3$ |
|---|---|---|
| $C_4H_9$-iso | H | 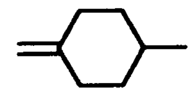 |
| $C_4H_9$-iso | H | 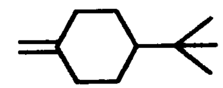 |
| $C_4H_9$-iso | H | $=CH-CH=CH-CH_3$ |
| $C_4H_9$-iso | H | $=CH-CH=C(CH_3)_2$ |
| $C_4H_9$-iso | H | $=CH-C_6H_5$ |
| $C_4H_9$-iso | H | $=CH-CH_2-C_6H_5$ |
| $C_4H_9$-iso | H | $=CH-CH_2CH_2C_6H_5$ |
| $C_4H_9$-iso | H | $=CH-CH=CH-C_6H_5$ |
| $C_4H_9$-iso | H | $=CH-C_6H_4-4F$ |
| $C_4H_9$-iso | H | $=CH-C_6H_4-4Cl$ |
| $C_4H_9$-iso | H | $=CH-C_6H_4-CF_3$ |
| $C_4H_9$-iso | H | $=CH-C_6H_4-CH_3$ |
| $C_4H_9$-iso | H | $=CH-C_6H_4-4OCH_3$ |
| $C_4H_9$-iso | H | $=CH-C_6H_4-2OH$ |
| $C_4H_9$-iso | H | $=CH-C_6H_3-2OH-3Cl$ |
| $C_4H_9$-iso | H | $=CH-C_6H_3-2OH-5Cl$ |
| $C_4H_9$-iso | H | =CH-(3-Pyridinyl) |
| $C_4H_9$-iso | H | $=CH-CH=CH-C_6H_4-4Cl$ |

Tabelle 1 (Fortsetzung)

| R | $R^1$ | $R^2$ | $R^3$ |
|---|---|---|---|
| $C_4H_9$-iso | $-CH_2-C_6H_5$ | H | H |
| $C_5H_{11}$-n | H | H | H |
| $C_4H_9$-iso | H | $-C_6H_4-4C(CH_3)_3$ | H |
| $C_4H_9$-iso | H | $-C_6H_4-4OCH_3$ | H |
| $C_4H_9$-iso | H | $-C_6H_4-4CF_3$ | H |
| $C_4H_9$-iso | H | $-C_6H_4-4NO_2$ | H |
| $C_4H_9$-iso | H | $-C_6H_4-4OC_2H_5$ | H |
| $C_5H_{11}$-n | $CH_3$ | H | H |
| $C_5H_{11}$-n | $CH_3$ | $CH_3$ | H |
| $C_5H_{11}$-n | iso-$C_3H_7$ | H | H |
| $C_5H_{11}$-n | H | $CH_3$ | $CH_3$ |
| $C_6H_{13}$-n | H | H | H |
| $C_6H_{13}$-n | $CH_3$ | H | H |
| $C_6H_{13}$-n | H | $CH_3$ | $CH_3$ |
| $C_6H_{13}$-n | H | $C_6H_5$ | H |
| $C_8H_{17}$-n | H | H | H |
| $C_8H_{17}$-n | $CH_3$ | H | H |
| $C_8H_{17}$-n | iso-$C_3H_7$ | H | H |
| $C_8H_{17}$-n | H | $C_6H_5$ | H |
| $C_{10}H_{21}$-n | H | H | H |
| $C_{10}H_{21}$-n | $CH_3$ | H | H |
| $C_{10}H_{21}$-n | iso-$C_3H_7$ | H | H |

Tabelle 1 (Fortsetzung)

| R | $R^1$ | $R^2$ | $R^3$ |
|---|---|---|---|
| $C_{10}H_{21}$-n | H | $CH_3$ | $CH_3$ |
| $C_{12}H_{25}$-n | H | H | H |
| $C_{12}H_{25}$-n | $CH_3$ | H | H |
| $C_{12}H_{25}$-n | H | $C_6H_5$ | H |
| $C_{14}H_{29}$-n | H | H | H |
| $C_{14}H_{29}$-n | iso-$C_3H_7$ | H | H |
| $C_{18}H_{37}$-n | H | H | H |
| $C_{20}H_{41}$-n | H | H | H |
| $Cl-CH_2-CH_2-$ | H | H | H |
| $Cl-(CH_2)_3-$ | H | H | H |
| $Cl-(CH_2)_4-$ | H | H | H |
| $Cl-(CH_2)_6-$ | H | H | H |
| $Cl-(CH_2)_8-$ | H | H | H |
| $Br-(CH_2)_4$ | H | H | H |
| $CF_3-CH_2-$ | H | H | H |
| $-CH_2-CH=CH_2$ | H | H | H |
| $-CH_2-CH=CH_2$ | H | $CH_3$ | $CH_3$ |
| $-CH_2-CH=CH_2$ | iso-$C_3H_7$ | H | H |
| $-CH-CH=CH_2$ | H | $C_6H_5-$ | H |
| $-CH_2-CH=CH-CH_3$ | H | H | H |
| $-CH_2-CH=C(CH_3)_2$ | H | H | H |
| $-CH_2CH_2CH=CH_2$ | H | H | H |

Tabelle 1 (Fortsetzung)

| R | $R^1$ | $R^2$ | $R^3$ |
|---|---|---|---|
| $-CH_2CH{=}CH-C_2H_5$ | H | H | H |
| $-CH_2CH{=}CH-C_3H_7-n$ | H | H | H |
| $-CH_2CH{=}CH-C_4H_9-n$ | H | H | H |
| $-CH_2-C(Cl){=}CH_2$ | H | H | H |
| $-CH_2-CH{=}CH-CL$ | H | H | H |
| $-CH_2-C(Br){=}CH_2$ | H | H | H |
| $-CH_2-CH{=}C(Cl)_2$ | H | H | H |
| $CH_2-(Cl){=}CCl_2$ | H | H | H |
| $-CH_2-CH_2-OCH_3$ | H | H | H |
| $-(CH_2)_3OCH_3$ | H | H | H |
| $-(CH_2)_2OC_2H_5$ | H | H | H |
| $-(CH_2)_2OC_3H_7-n$ | H | H | H |
| $-(CH_2)_3OC_2H_5$ | H | H | H |
| $-(CH_2)_3OC_3H_7-n$ | H | H | H |
| $-(CH_3)_3OC_4H_9-n$ | H | H | H |
| -Cyclopropyl | H | H | H |
| -Cyclobutyl | H | H | H |
| -Cyclopentyl | H | H | H |
| -Cyclopentylmethyl | H | H | H |
| -Cyclohexyl | H | H | H |
| 4-Methylcyclohexyl | H | H | H |
| Cyclohexylmethyl | H | H | H |

Tabelle 1 (Fortsetzung)

| R | $R^1$ | $R^2$ | $R^3$ |
|---|---|---|---|
| Cycloheptyl | H | H | H |
| $-CH_2-C_6H_5$ | H | H | H |
| $-CH_2CH_2C_6H_5$ | H | H | H |
| $-(CH_2)_3C_6H_5$ | H | H | H |
| $-CH_2CH(CH_3)CH_2C_6H_5$ | H | H | H |
| $-CH_2-CH=CH-C_6H_5$ | H | H | H |
| $-CH_2-C_6H_4-2F$ | H | H | H |
| $-CH_2-C_6H_4-4F$ | H | H | H |
| $-CH_2-C_6H_4-2Cl$ | H | H | H |
| $-CH_2-C_6H_4-3Cl$ | H | H | H |
| $-CH_2-C_6H_4-4Cl$ | H | H | H |
| $-CH_2-C_6H_4-4CH_3$ | H | H | H |
| $-CH_2-C_6H_4-2OCH_3$ | H | H | H |
| $-CH_2-C_6H_4-3OCH_3$ | H | H | H |
| $-CH_2-C_6H_4-4OCH_3$ | H | H | H |
| $-CH_2-C_6H_4-4OC_2H_5$ | H | H | H |
| $-CH_2-C_6H_4-4CF_3$ | H | H | H |
| $-CH_2-C_6H_3-2,4-Cl_2$ | H | H | H |
| $-(CH_2)_4-C_6H_4-4Cl$ | H | H | H |

Die Herstellung und die Verwendung der neuen Wirkstoffe der allgemeinen Formel I geht aus den nachfolgenden Beispielen hervor.

Herstellungsbeispiele

Beispiel 1

1-(Ethoxyamino)-2-(hydrazino)-ethan-1,2-dion

Die Lösung von 92,8 g (1,855 Mol) Hydrazinhydrat in 300 ml Ethanol wurde mit einer Lösung von 234 g (1,59 Mol) (Ethoxyamino)-oxoessigsäuremethylester in 450 ml Ethanol versetzt und 2 Stunden bei 65 °C gerührt. Das Gemisch wurde auf +10 °C abgekühlt, der Niederschlag abgesaugt, mit wenig kaltem Ethanol gewaschen und getrocknet. Man erhielt 150g (64,2 % d. Theorie) 1-(Ethoxyamino)-2-(hydrazino)-ethan-1,2-dion als weiße Kristalle vom Schmp. 182 °C.

Beispiel 2

1-(Ethoxyamino)-2-[(4-chlorphenyl-methylen)-hydrazino]-ethan-1,2-dion

Eine Lösung von 10,3 g (0,07 Mol) 1-(Ethoxyamino)-2-(hydrazino)-ethan-1,2-dion (Beispiel 1) in 100 ml Essigsäure wurde mit 9 g (0,07 Mol) 4-Chlorbenzaldehyd versetzt und 30 Minuten bei 40 ° C gerührt. Der Niederschlag wurde abgesaugt, mit Ether gewaschen und getrocknet. Man erhielt 12,2 g (65 % d. Theorie) 1-(Ethoxyamino)-2-[4-chlorphenyl-methylen)-hydrazino]-ethan-1,2-dion als weiße Kristalle- vom Schmp. 330 ° C (Zers.).

Analog zur den Herstellungsbeispielen und gemäß den allgemeinen Angaben zur Herstellung erhielt man die in Tabelle 2 aufgeführten Verbindungen der allgemeinen Formel I:

Tabelle 2

| Nr. | R | $R^1$ | $R^2$ | $R^3$ | Schmp. (°C) |
|---|---|---|---|---|---|
| 3 | $-C_2H_5$ | H | 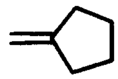 | | 130-131 |
| 4 | $-C_2H_5$ | H | 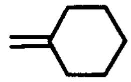 | | 123-125 |
| 5 | $-C_2H_5$ | H | 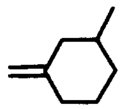 | | 110-112 |
| 6 | $-C_2H_5$ | H | 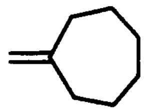 | | 120-122 |
| 7 | $-CH_2-CH=CH_2$ | H | H | H | 185-186 |
| 8 | $-CH_2-CH=CH_2$ | H | $=CH-C_6H_4-4Cl$ | | 300 (Zers.) |
| 9 | $-CH_2-C(Br)=CH_2$ | H | H | H | 130-131 |
| 10 | $C_3H_7$-n | H | H | H | 159-161 |
| 11 | $C_3H_7$-n | H | $=CH-C_6H_4-4Cl$ | | 258-259 |
| 12 | $C_3H_7$-n | H | $=CH-C_6H_4-4OCH_3$ | | 199-201 |
| 13 | $C_3H_7$-n | H | $=CH-C_6H_3-3,4(OCH_3)_2$ | | 209-211 |
| 14 | $C_3H_7$-iso | H | H | H | 142-145 |
| 15 | $C_3H_7$-iso | H | $=CH-C_6H_4-4OCH_3$ | | 243-245 |
| 16 | $C_3H_7$-iso | H | $=CH-C_6H_3-3,4(OCH_3)_2$ | | 198-201 |
| 17 | $C_3H_7$-iso | H | $=CH-C_6H_2-3,4,5(OCH_3)_3$ | | 235-238 |
| 18 | $C_3H_7$-iso | H | 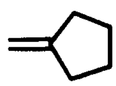 | | 158-159 |
| 19 | $C_3H_7$-iso | H | 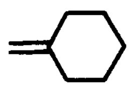 | | 138-140 |
| 20 | $C_3H_7$-iso | H | 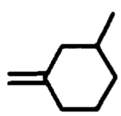 | | 117-118 |
| 21 | $C_3H_7$-iso | H | 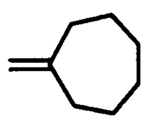 | | 120-121 |
| 22 | $-CH_2-C(CH_3)=CH_2$ | H | H | H | 142-144 |
| 23 | $-CH_2-C(CH_3)=CH_2$ | H | $=CH-C_6H_4-4Cl$ | | 277-280 |
| 24 | $-CH_2-C(CH_3)=CH_2$ | H | $=CH-C_6H_4-4CF_3$ | | 310 (Zers.) |
| 25 | $-CH_2-C(CH_3)=CH_2$ | H | $=CH-C_6H_4-4OCH_3$ | | 300 (Zers.) |

Tabelle 2 (Fortsetzung)

| Nr. | R | $R^1$ | $R^2$ | $R^3$ | Schmp. (°C) |
|---|---|---|---|---|---|
| 19 | $C_3H_7$-iso | H | 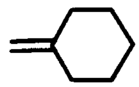 | | 138-140 |
| 26 | $-CH_2-C(CH_3)=CH_2$ | H | $=CH-C_6H_3-3,4(OCH_3)_2$ | | 205-208 |
| 27 | $C_4H_9$-n | H | H | H | 150-151 |
| 28 | $C_4H_9$-n | H | $=CH-C_6H_4-4OCH_3$ | | 217-220 |
| 29 | $C_4H_9$-n | H | $=CH-C_6H_3-3,4(OCH_3)_2$ | | 217-219 |
| 30 | $C_4H_9$-n | H | $=CH-C_6H_3-3,4Cl_2$ | | 203-205 |
| 31 | $C_4H_9$-iso | H | H | H | 151-153 |
| 32 | $C_4H_9$-iso | $CH_3$ | H | H | 130-131 |
| 33 | $C_4H_9$-iso | H | $CH_3$ | $CH_3$ | 107-111 |
| 34 | $C_4H_9$-iso | $C_3H_7$-iso | H | H | 135-136 |
| 35 | $C_4H_9$-iso | H | $=CH-C_6H_5$ | | 143-146 |
| 36 | $C_4H_9$-iso | H | $=CH-C_6H_4-2OH$ | | 218-220 |
| 37 | $C_4H_9$-iso | H | $=CH-C_6H_4-4C(CH_3)_3$ | | 275-277 |
| 38 | $C_4H_9$-iso | H | $=CH-C_6H_4-4OCH_3$ | | 230 |
| 39 | $C_4H_9$-iso | H | $=CH-C_6H_3-3,4(OCH_3)_2$ | | 212-215 |
| 40 | $C_4H_9$-iso | H | $=CH-C_6H_3-2OH-5Cl$ | | 238-240 |
| 41 | $C_4H_9$-iso | H | 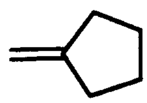 | | 153-155 |
| 42 | $C_4H_9$-iso | H | 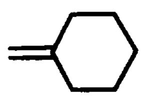 | | 122-125 |
| 43 | $C_4H_9$-iso | H | 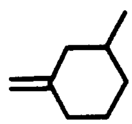 | | 112-115 |
| 44 | $C_4H_9$-iso | $CH_3$ | 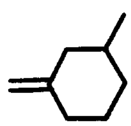 | | 157-158 |
| 45 | $C_4H_9$-iso | H | 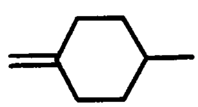 | | 105-108 |
| 46 | $C_4H_9$-iso | H | 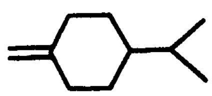 | | 112-114 |

Tabelle 2 (Fortsetzung)

| Nr. | R | $R^1$ | $R^2$ | $R^3$ | Schmp. (°C) |
|---|---|---|---|---|---|
| 47 | $C_4H_9$-iso | H | 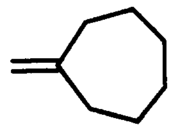 | | 154-156 |
| 48 | $-CH_2-C_6H_5$ | H | H | H | 156-158 |
| 49 | $-CH_2-C_6H_5$ | H | $=CH-C_6H_4-4Cl$ | | 274-278 |
| 50 | $-CH_2-C_6H_5$ | H | $=CH-C_6H_4-4OCH_3$ | | 249-250 |
| 51 | $-CH_2-C_6H_5$ | H | $=CH-C_6H_3-3,4(OCH_3)_2$ | | 240-241 |
| 52 | $C_2H_5$ | H | 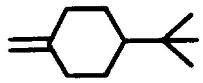 | | Harz |
| 53 | $C_3H_7$-iso | H | 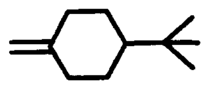 | | 117-120 |

Die neuen Verbindungen zeichnen sich, allgemein ausgedrückt, durch eine hervorragende Wirksamkeit gegen ein breites Spektrum von pflanzenpathogenen Pilzen, insbesondere aus der Klasee der Ascomyceten und Basidiomyceten, aus. Sie sind zum Teil systemisch wirksam und können als Blatt- und Bodenfungizide eingesetzt werden.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis, Mais, Rasen, Baumwolle, Soja, Kaffee, Zuckerrohr, Obst und Zierpflanzen im Gartenbau, Weinbau sowie Gemüse - wie Gurken, Bohnen und Kürbisgewächse -.

Die neuen Verbindungen sind insbesondere geeignet zur Bekämpfung folgender Pflanzenkrankheiten:
Erysiphe graminis (echter Mehltau) in Getreide,
Erysiphe cichoracearum und Sphaerotheca fuliginea an Kürbisgewächsen,
Podosphaera leucotricha an Äpfeln,
Uncinula necator an Reben,
Puccinia-Arten an Getreide,
Rhizoctonia-Arten an Baumwolle und Rasen,
Ustilago-Arten an Getreide und Zuckerrohr,
Venturia inaequalis (Schorf) an Äpfeln,
Helminthosporium-Arten an Getreide,
Septoria nodorum an Weizen,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,
Phytophthora infestans an Kartoffeln und Tomaten,
Fusarium- und Verticillium-Arten an verschiedenen Pflanzen,
Plasmopara viticola an Reben,
Alternaria-Arten an Gemüse und Obst.

Die Verbindungen werden angewendet, indem man die Pflanzen mit den Wirkstoffen besprüht und bestäubt oder die Samen der Pflanzen mit den Wirkstoffen behandelt. Die Anwendung erfolgt vor oder nach der Infektion der Pflanzen oder Samen durch die Pilze.

Die neuen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanz gewährleisten. Die Formulierungen werden in bekannter Weise hergestellt, z. B. durch

Verstrecken des Wirkstoffs mit Lösungsmitteln und/oder Trägerstoffen, gegebenenfalls unter Verwendung von Emulgierstoffen und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z. B. Xylol), chlorierte Aromaten (z. B. Chlorbenzole), Paraffine (z. B. Erdölfraktionen), Alkohole (z. B. Methanol, Butanol), Ketone (z. B. Cyclohexanon), Amine (z. B. Ethanolamin, Dimethylformamid) und Wasser; Trägerstoffe wie natürliche Gesteinsmehle (z. B. Kaoline, Tonerden, Talkum, Kreide) und synthetische Gesteinsmehle (z. B. hochdisperse Kieselsäure, Silikate); Emulgiermittel, wie nichtionogene und anionische Emulgatoren (z. B. Polyoxyethylen-Fettalkohol-Ether, Alkylsulfonate und Arylsulfonate) und Dispergiermittel wie Lignin, Sulfitablaugen und Methylcellulose.

Die fungiziden Mittel enthalten im allgemeinen zwischen 0,1 und 95, vorzugsweise zwischen 0,5 und 90 Gew.-% Wirkstoff.

Die Aufwandmengen liegen je nach Art des gewünschten Effektes zwischen 0,02 und 3 kg Wirkstoff oder mehr je ha. Die neuen Verbindungen können auch im Materialschutz (Holzschutz) eingesetzt werden, z. B. gegen Paecilomyces variotii.

Bei der Saatgutbehandlung werden im allgemeinen Wirkstoffmengen von 0,001 bis 50 g, vorzugsweise 0,01 bis 10 g Wirkstoff je Kilogramm Saatgut benötigt.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweise durch Versprühen, Vernebeln, Verstäuben, Verstreuen, Beizen oder Gießen.

Beispiele für solche Zubereitungen sind:

I Man vermischt 90 Gew.-Teile der Verbindung gemäß Beispiel 1 mit 10 Gew.-Teilen N-Methyl-$\alpha$-pyrrolidon und erhält eine Lösung, die zur Anwendung in Form kleinster Tropfen geeignet ist.

II 20 Gew.-Teile der Verbindung gemäß Beispiel 2 werden in einer Mischung gelöst, die aus 80 Gew.-Teilen Xylol, 10 Gew.-Teilen des Anlagerungsproduktes von 8 bis 10 Mol Ethylenoxid an 1Mol Ölsäure-N-monoethanolamid, 5 Gew.-Teilen Calciumsalz der Dodecylbenzolsulfonsäure und 5 Gew.-Teilen des Anlagerungsproduktes und 40 Mol Ethylenoxid an 1 Mol Ricinusöl besteht. Durch Ausgießen und feines Verteilen der Lösung in Wasser erhält man eine wäßrige Dispersion.

III 20 Gew.-Teile der Verbindung Nr. 3 aus Tabelle 2 werden in einer Mischung gelöst, die aus 40 Gew.-Teilen Cyclohexanon, 30 Gew.-Teilen Isobutanol, 20 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

IV 20 Gew.-Teile der Verbindung Nr. 4 aus Tabelle 2 werden in einer Mischung gelöst, die aus 25 Gew.-Teilen Cyclohexanol, 65 Gew.-Teilen einer Mineralölfraktion vom Siedepunkt 210 bis 280 °C und 10 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

V 80 Gew.-Teile der Verbindung Nr. 5 aus Tabelle 2 werden mit 3 Gew.-Teilen des Natriumsalzes der Diisobutylnaphthalin-$\alpha$-sulfonsäure, 10 Gew.-Teilen des Natriumsalzes einer Ligninsulfonsäure aus einer Sulfitablauge und 7 Gew.-Teilen pulverförmigem Kieselsäuregel gut vermischt und in einer Hammermühle vermahlen. Durch feines Verteilen der Mischung in Wasser erhält man eine Spritzbrühe.

VI 3 Gew.-Teile der Verbindung Nr. 6 aus Tabelle 2 werden mit 97 Gew.-Teilen feinteiligem Kaolin innig vermischt. Man erhält auf diese Weise ein Stäubemittel, das 3 Gew.-% des Wirkstoffs enthält.

VII 30 Gew.-Teile der Verbindung Nr. 7 aus Tabelle 2 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII 40 Gew.-Teile der Verbindung Nr. 8 aus Tabelle 2 werden mit 10 Gew.-Teilen Natriumsalz eines Phenolsulfonsäureharnstoff-formaldehyd-Kondensats, 2 Gew.-Teilen Kieselgel und 48 Gew.-Teilen Wasser innig vermischt. Man erhält eine stabile wäßrige Dispersion. Durch Verdünnen mit Wasser erhält man eine wäßrige Dispersion.

IX 20 Gew.-Teile der Verbindung Nr. 9 aus Tabelle 2 werden mit 2 Gew.-Teilen Calciumsalz der Dodecylbenzolsulfonsäure, 8 Gew.-Teilen Fettalkoholpolyglykolether, 2 Gew.-Teilen Natriumsalz eines Phenolsulfonsäureharnstoff-formaldehyd-Kondensats und 68 Gew.-Teilen eines paraffinischen Mineralöls innig vermischt. Man erhält eine stabile ölige Dispersion.

Die erfindungsgemäßen Mittel können in diesen Anwendungsformen auch zusammen mit anderen Wirkstoffen vorliegen, wie z. B. Herbiziden, Insektiziden, Wachstumsregulatoren und Fungiziden, oder auch mit Düngemitteln vermischt und ausgebracht werden. Beim Vermischen mit Fungiziden erhält man dabei in vielen Fällen eine Vergrößerung des fungiziden Wirkungsspektrums.

Für die folgenden Vergleichsversuche wurde der bekannte Wirkstoff Tridemorph (A) - bekannt aus US 3 686 394 - verwendet.

Anwendungsbeispiel 1

Wirksamkeit gegen Fusarium culmorum an Weizen

Primär-Blätter von in Töpfen gewachsenem Weizen der Sorte "Kanzler" wurden mit wäßriger Spritzbrühe, die 80 % Wirkstoff und 20 % Emulgiermittel in der Trockensubstanz enthielt, tropfnaß bespritzt. Am folgenden Tage wurden sie mit einer Sporensuspension von Fusarium culmorum inokuliert und anschließend in eine Klimakammer mit hoher Luftfeuchtigkeit (mehr als 90 %) bei 22-24 °C gestellt. Nach 6 Tagen wurde das Ausmaß der Symptomentwicklung visuell ausgewertet.

Das Ergebnis des Versuchs zeigt, daß bei der Anwendung einer 500 ppm Wirkstoff enthaltenden Spritzbrühe die Verbindungen Nr. 9, 10, 14, 27, 31, 35, 41, 45, 46 und 47 eine sehr gute fungizide Wirkung zeigen (10 % Befall).

Anwendungsbeispiel 2

Wirksamkeit gegen Botrytis cinerea

Paprikasämlinge der Sorte "Neusiedler Ideal Elite" wurden, nachdem sich 4-5 Blätter gut entwickelt hatten, mit wäßrigen Suspensionen, die 80 % Wirkstoff und 20 % Emulgiermittel in der Trokkensubstanz enthielten, tropfnaß gespritzt. Nach dem Antrocknen des Spritzbelages wurden die Pflanzen mit einer Konidienaufschwemmung des Pilzes Botrytis cinerea besprüht und bei 22-24 °C in eine Kammer mit hoher Luftfeuchtigkeit gestellt. Nach 5 Tagen hatte sich die Krankheit auf den unbehandelten Kontrollpflanzen so stark entwickelt, daß die entstandenen Blattnekrosen den überwiegenden Teil der Blätter bedecken.

Das Ergebnis des Versuchs zeigt, daß bei der Anwendung einer 500 ppm Wirkstoff enthaltenden Spritzbrühe die Verbindungen Nr. 7, 22, 24, 26, 31, 42, 45 und 46 eine bessere fungizide Wirkung zeigen (10 % Befall) als der bekannte Wirkstoff A (60 % Befall).

## Patentansprüche

**1.** Oxalylhydrazid-hydroxamsäure-Derivate der allgemeinen Formel I

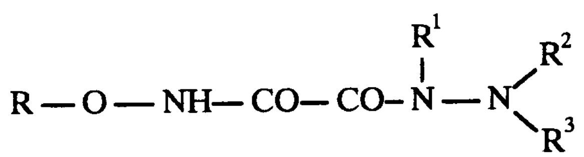

I

in der die Sustituenten folgende Bedeutung haben:

R, $R^1$, $R^2$ und $R^3$ unabhängig voneinander

Wasserstoff, $C_1$-$C_{20}$-Alkyl, $C_3$-$C_{18}$-Alkenyl oder $C_3$-$C_8$-Alkinyl, wobei diese Reste ein bis fünf Halogenatome oder ein bis drei $C_1$-$C_4$-Alkoxygruppen, gegebenenfalls zusammen mit den Halogenatomen, tragen können;

monocyclisches oder polycyclisches $C_3$-$C_{10}$-Cycloalkyl oder $C_3$-$C_{10}$-Cycloalkylmethyl, wobei diese Ringe ein bis drei $C_1$-$C_4$-Alkylgruppen oder einen Cyclohexylring tragen können;

monocyclisches oder polycyclisches $C_5$-$C_{10}$-Cycloalkenyl oder $C_5$-$C_{10}$-Cycloalkenylmethyl, wobei diese Ringe ein bis drei $C_1$-$C_4$-Alkylgruppen oder einen Cyclohexylring tragen können;

Phenyl, Phenyl-$C_1$-$C_4$-alkyl, Phenyl-$C_2$-$C_6$-alkenyl, einkernige Heteroaryl- oder Heteroaryl-$C_1$-$C_4$-alkylreste, wobei die (hetero)-aromatischen Reste ein bis fünf Halogenatome oder ein bis drei der folgenden Gruppen, gegebenenfalls zusammen mit den Halogenatomen tragen können: Hydroxy, Nitro, $C_1$-$C_4$-Alkyl, $C_1$-$C_4$-Halogenalkyl, $C_1$-$C_4$-Alkoxy, $C_1$-$C_4$-Halogenalkoxy, COOR, $CONH_2$ oder Amine, wobei $R^2$ und $R^3$ auch zusammen mit dem Stickstoffatom, dessen Substituenten sie sind, einen 5- oder 6-gliedrigen Ring bilden können, der noch durch Sauerstoff unterbrochen sein kann, oder

$R^2$ zusammen mit $R^3$ für die Gruppierung

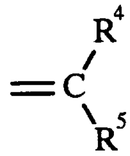

stehen, wobei $R^4$ und $R^5$ für einen der Reste R, $R^1$, $R^2$ oder $R^3$ stehen oder $R^4$ zusammen mit $R^5$ und dem C-Atom, dessen Substituenten sie sind, für monocyclisches oder polycyclisches $C_3$-$C_{10}$-Cycloalkyl steht, wobei diese Ringe ein bis drei $C_1$-$C_4$-Alkylgruppen oder einen Phenylring tragen können oder $R^4$ zusammen mit $R^5$ und dem C-Atom, dessen Substituenten sie sind, einen 5- oder 6-gliedrigen, gesättigten Heterocyclus mit O, S oder -N($R^6$)- als Heterogruppierung bilden, wobei $R^6$ für eine $C_1$-$C_6$-Alkylgruppe steht, oder mit zwei nicht benachbarten gleichen oder verschiedenen Heteroatomen, die Sauerstoff, Schwefel oder die Gruppierung -N($R^6$) sein können, als Heterogruppierung bilden.

**2.** Verfahren zur Herstellung von Oxalylhydrazid-hydroxamsäureDerivaten der Formel I gemäß Anspruch I, dadurch gekennzeichnet, daß man

a) Hydrazine der Formel II

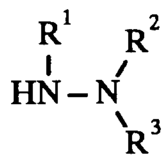

(II)

mit einem Oxalyl-hydroxamester der Formel III

R-O-NH-CO-$COOR^6$ (III)

wobei R, $R^1$, $R^2$, $R^3$ und $R^6$ die oben genannte Bedeutung haben, oder

b) ein Oxalylhydrazid-hydrdoxamsäureester der Formel IV

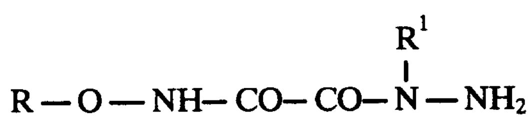

(IV)

mit einem Aldehyd oder Keton der Formel V

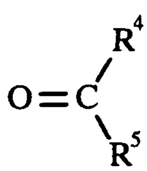

(V)

wobei R, $R^1$, $R^4$ und $R^5$ die oben angegebenen Bedeutungen haben, gegebenenfalls in Gegenwart eines Lösungs- oder Verdünnungsmittels oder einer anorganischen oder organischen Base oder einer anorganischen oder organischen Säure oder eines Reaktionsbeschleunigers oder deren Mischungen umsetzt.

**3.** Verfahren zur Bekämpfung von Pilzen, dadurch gekennzeichnet, daß man die Pilze oder die von Pilzbefall bedrohten Materialien, Pflanzen, Saatgüter oder den Erdboden mit einer fungizid wirksamen Menge eines Oxalylhydrazid-hydroxamsäure-Derivats der Formel I behandelt.

**4.** Fungizid, enthaltend einen inerten Trägerstoff und eine fungizid wirksame Menge eines Oxalylhydrazid-hydroxamsäure-Derivats der Formel I.

**5.** Verfahren zur Herstellung eines funigiziden Mittels, dadurch gekennzeichnet, daß man eine oder mehrere Verbindungen der Formel I gemäß Anspruch 1 mit einem festen oder flüssigen Trägerstoff sowie gegebenenfalls mit einem oder mehreren oberflächenaktiven Mitteln mischt.

**6.** Verbindung der Formel I gemäß Anspruch 1, in der R Ethyl, $R^1$, $R^2$ und $R^3$ Wasserstoff bedeuten.

**7.** Verbindung der Formel I gemäß Anspruch 1, in der R Ethyl, $R^1$ Wasserstoff, $R^2$ und $R^3$ zusammen 4-Chlorphenylmethylen bedeuten.

**8.** Verbindung der Formel I gemäß Anspruch 1, in der R Ethyl, $R^1$ Wasserstoff, $R^2$ und $R^3$ zusammen Cyclopentylen bedeuten.

**9.** Verbindung der Formel I gemäß Anspruch 1, in der R Ethyl, $R^1$ Wasserstoff, $R^2$ und $R^3$ zusammen Cyclohexylen bedeuten.

**10.** Verbindung der Formel I gemäß Anspruch 1, in der R Ethyl, $R^1$ Wasserstoff, $R^2$ und $R^3$ zusammen 3-Methylcyclohexylen bedeuten.

Europäisches Patentamt

# EUROPÄISCHER RECHERCHENBERICHT

Nummer der Anmeldung
EP 93 11 9945

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.5) |
|---|---|---|---|
| X | CHEMICAL ABSTRACTS, vol. 89, no. 13, 25. September 1978, Columbus, Ohio, US; abstract no. 108560j, PETYUNIN, G. P. ET. AL. 'Amides and hydrazides of oxalic acid. XXXVII. Synthesis and biological activity of substituted carbamidohydroxamic acids.' Seite 830 ;Spalte 2 ; * Zusammenfassung * & KHIM.-FARM. ZH. Bd. 12, Nr. 6 , 1978 Seiten 106 - 8 | 1-5 | C07C259/06 A01N37/30 |
| A | EP-A-0 465 986 (B.A.S.F.) * Ansprüche; Beispiele * | 1-10 | |
| A | EP-A-0 469 423 (B.A.S.F.) * Ansprüche; Beispiele * | 1-10 | |
| | | | RECHERCHIERTE SACHGEBIETE (Int.Cl.5) |
| | | | C07C |

Der vorliegende Recherchenbericht wurde für alle Patentansprüche erstellt

| Recherchenort | Abschlußdatum der Recherche | Prüfer |
|---|---|---|
| DEN HAAG | 21. März 1994 | Helps, I |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P04C03)